# DEMANDE DE BREVET EUROPEEN

(11) **EP 1 486 502 A1**
(43) Date de publication de la demande: **15.12.2004**
(21) Numéro de dépôt: 04291434.1
(22) Date de dépôt: 09.06.2004
(51) Int. Cl.: C07D 513/04, A61K 31/54, A61P 25/28

(54) **Dérivés de benzothiazine et benzothiadiazine, leur procédé de préparation et les compositions pharmaceutiques qui les contiennent**

(30) Priorité: 13.06.2003 FR 0307117
(71) Demandeur: LES LABORATOIRES SERVIER, 92415 Courbevoie Cédex (FR)
(72) Inventeur: Desos, Patrice, 92270 Bois-Colombes (FR); Cordi, Alex, 92150 Suresnes (FR); Lestage, Pierre, 78170 La Celle Saint Cloud (FR)

(57) **Abrégé**

Composés de formule (I) : 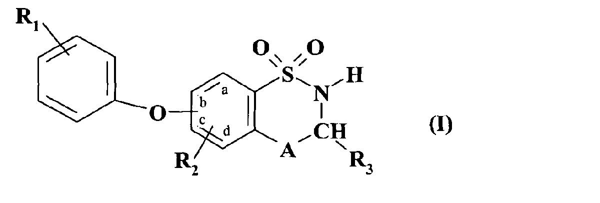 dans laquelle :
**R**_{**1**} représente un hétérocycle,
**R**_{**2**} représente un hydrogène, halogène ou un hydroxy,
**A** représente CR₄R₅ ou NR₄,
**R**_{**3**} représente un hydrogène, alkyle ou cycloalkyle,
**R**_{**4**} représente un hydrogène ou un alkyle,
ou bien
**A** représente un azote et forme avec -CHR₃- adiacent le cycle 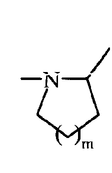 dans lequel m représente 1, 2 ou 3,
**R**_{**5**} représente un hydrogène ou halogène,
leurs isomères ainsi que leurs sels d'addition.
Médicaments.

## Description

La présente invention concerne de nouveaux dérivés de benzothiazine et benzothiadiazine, leur procédé de préparation et les compositions pharmaceutiques qui les contiennent.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39, 517-545).

Par ailleurs, d'innombrables travaux ont démontré durant les dernières années l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits très récemment comme modulant positivement les récepteurs AMPA des cellules neuronales (Journal of Neurochemistry, 1992, 58, 1199-1204).

Dans la littérature, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Enfin, le brevet EP 692 484 décrit un dérivé de benzothiadiazine possédant une activité facilitatrice sur le courant AMPA et la demande de brevet WO 99/42456 décrit entre autres certains dérivés de benzothiadiazine en tant que modulateurs des récepteurs AMPA.

Les dérivés de benzothiazine et de benzothiadiazine, objets de la présente invention, outre le fait qu'ils soient nouveaux, présentent, de manière surprenante, des activités pharmacologiques sur le courant AMPA nettement supérieures à celles des composés de structures proches décrits dans l'Art Antérieur. Ils sont utiles en tant que modulateurs AMPA pour le traitement ou la prévention des désordres mnémocognitifs associés à l'âge, aux syndromes anxieux ou dépressifs, aux maladies neurodégénératives progressives, à la maladie d'Alzheimer, à la maladie de Pick, à la chorée d'Huntington, à la schizophrénie, aux séquelles des maladies neurodégénératives aiguës, aux séquelles de l'ischémie et aux séquelles de l'épilepsie.

Plus spécifiquement, la présente invention concerne les composés de formule **(I)** : 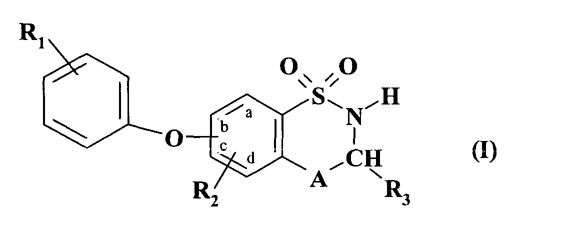 dans laquelle :
- **R**_{**1**}: représente un groupement hétérocyclique,
- **R**_{**2**}: représente un atome d'hydrogène, d'halogène ou un groupement hydroxy,
- **A**: représente un groupement CR₄R₅ ou un groupement NR₄,
- **R**_{**3**}: représente un atome d'hydrogène, un groupement alkyle (C₁-C₆) linéaire ou ramifié ou un groupement cycloalkyle (C₃-C₇),
- **R**_{**4**}: représente un atome d'hydrogène ou un groupement alkyle (C₁-C₆) linéaire ou ramifié,
ou bien
- **A**: représente un atome d'azote et forme avec le groupement -CHR₃- adjacent le cycle 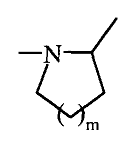 dans lequel m représente 1, 2 ou 3,
- **R**_{**5**}: représente un atome d'hydrogène ou d'halogène,
leurs énantiomères et diastéréoisomères ainsi que leurs sels d'addition à un acide ou à une base pharmaceutiquement acceptable,
étant entendu que par groupement hétérocyclique, on comprend groupement aromatique ou non, monocyclique ou bicyclique, contenant un à quatre hétéroatomes, identiques ou différents, choisis parmi azote, oxygène et soufre, éventuellement substitué par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, alkoxycarbonyle (C₁-C₆) linéaire ou ramifié, oxo, thioxo, carboxy, acyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié), ou alkyl (C₁-C₆) sulfonylamino.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Le groupement 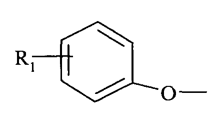 est préférentiellement en position b du phényle qui le porte.

R₁ représente préférentiellement un groupement hétérocyclique monocyclique.

Plus préférentiellement, R₁ représente un groupement hétérocyclique monocyclique aromatique contenant 1 à 4 hétéroatomes choisis parmi azote, soufre et oxygène, et plus particulièrement azote et oxygène comme par exemple les groupements tétrazolyle, triazolyle, imidazolyle, pyrazolyle, pyrrolyle, pyridinyle, furanyle, oxazolyle, oxadiazolyle.

R₁ représente également avantageusement un groupement hétérocyclique monocyclique contenant 1 à 4 hétéroatomes choisis parmi oxygène, soufre et azote, et plus particulièrement les groupements oxathiadiazolyle, dihydrooxadiazolyle, morpholinyle.

Avantageusement, le groupement R₁ est non substitué ou substitué par un groupement alkyle (C₁-C₆) linéaire ou ramifié comme le groupement méthyle par exemple, ou par un groupement oxo ou thioxo.

Le groupement R₁ est préférentiellement en position méta ou para du noyau phénoxy qui le porte.

Le groupement R₂ préféré est l'atome d'hydrogène.

Les composés préférés de l'invention sont les composés tels que A représente un atome d'azote et forme avec le groupement -CHR₃- adjacent le cycle 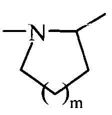 dans lequel m représente 1, 2 ou 3 et préférentiellement 1.

Les composés préférés de l'invention sont le 7-[3-(1*H*-tétrazol-5-yl)phénoxy]-2,3,3a,4-tétrahydro-1*H*-pyrrolo[2,1-*c*][1,2,4]benzothiadiazine-5,5-dioxide, le 7-[3-(3-furyl)phénoxy]-2,3,3a,4-tétrahydro-1*H*-pyrrolo[2,1-*c*][1,2,4]benzothiadiazine 5,5-dioxide et le 7-[3-(1,3-oxazol-5-yl)phénoxy]-2,3,3a,4-tétrahydro-1*H*-pyrrolo[2,1-*c*][1,2,4] benzothiadiazine.

Les énantiomères, diastéréoisomères ainsi que les sels d'addition à une base ou à un acide pharmaceutiquement acceptable des composés préférés de l'invention font partie intégrante de l'invention.

L'invention s'étend également au procédé de préparation des composés de formule (I).

Le procédé de préparation des composés de formule (I) dans laquelle A représente un groupement NR₄ ou A représente un atome d'azote et forme avec le groupement CHR₃ adjacent le cycle 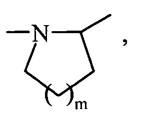 dans lequel m représente 1, 2 ou 3, est caractérisé en ce que l'on utilise comme produit de départ un composé de formule **(II) :** 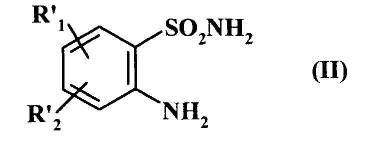 dans laquelle :
R'₁ représente un groupement alkoxy (C₁-C₆) linéaire ou ramifié,
R'₂ représente un atome d'hydrogène, d'halogène ou un groupement alkoxy (C₁-C₆) linéaire ou ramifié,
qui est :
**(a) soit mis en réaction** avec le chlorure d'acide de formule **(III)** en présence d'une base, en milieu tétrahydrofurane ou acétonitrile :

   **Cl - CH**_{**2**} **- (CH**_{**2**}**)**_{**m**} **- CH**_{**2**} **- COCl (III)**

   dans laquelle m a la même signification que dans la formule (I),
   pour conduire au composé de formule **(IV)** : 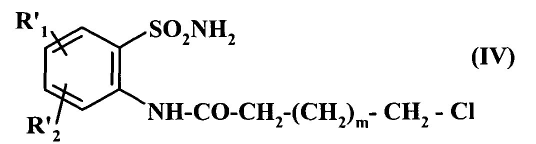 dans laquelle R'₁ et R'₂ sont tels que définis précédemment,
   qui est alors cyclisé en milieu basique, pour conduire au composé de formule (V) : 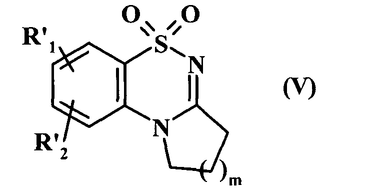 dans laquelle R'₁, R'₂ et m sont tels que définis précédemment,
   qui subit éventuellement une réduction, en milieu alcoolique ou diméthylformamide, en présence de borohydrure de sodium, pour conduire au composé de formule **(VI) :** 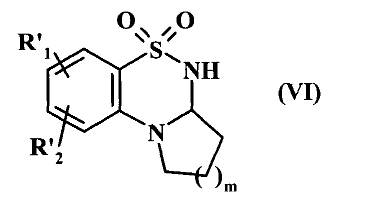 dans laquelle R'₁, R'₂ et m sont tels que définis précédemment,
   composé de formule **(V)** ou **(VI)** qui subit l'action du tribromure de bore,
   pour conduire au composé de formule **(VII) :** 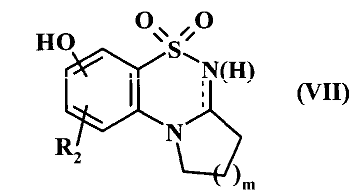 dans laquelle R₂ est tel que défini dans la formule (I) et m est tel que défini précédemment,
**(b) soit cyclisé :**
   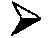 en présence d'une amidine de formule **(VIII)** : 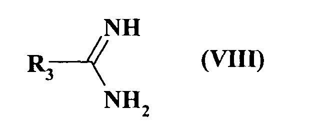 dans laquelle :
      R₃ est tel que défini dans la formule (I),
      pour conduire au composé de formule **(IX) :** 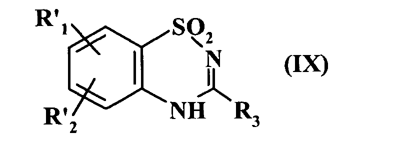 dans laquelle R'₁, R'₂, et R₃ ont la même signification que précédemment,
      qui est :
      ◆ ou bien réduit par un hydrure métallique,
         pour conduire au composé de formule **(X) :** 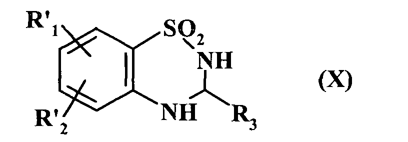
      dans laquelle R'₁, R'₂, et R₃ ont la même signification que précédemment,
      ◆ ou bien alkylé par action d'une base forte en présence d'un agent d'alkylation R'₄X dans lequel R'₄ représente un groupement alkyle (C₁-C₆) linéaire ou ramifié et X un atome d'halogène, puis réduit,
         pour conduire au composé de formule **(XI) :** 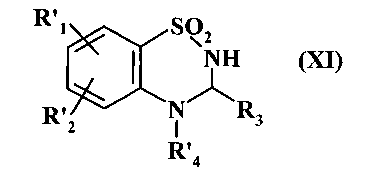
      dans laquelle R'₁, R'₂, R₃ et R'₄ ont la même signification que précédemment,
   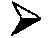 en présence d'un aldéhyde de formule **(XII) :** 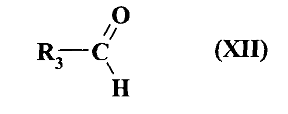 dans laquelle R₃ est tel que défini dans la formule (I),
      pour conduire au composé de formule (X) décrit précédemment,
      composé de formule (X) ou (XI),
      dont on transforme le groupement R'₁ et le groupement R'₂ lorsque celui-ci représente un groupement alkoxy (C₁-C₆) linéaire ou ramifié, en groupements hydroxy,
      pour conduire au composé de formule **(XIII) :** 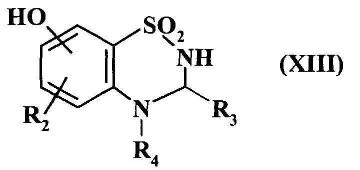 dans laquelle R₂, R₃ et R₄ ont la même signification que dans la formule (I),
      composé de formule (VII) ou (XIII) que l'on fait réagir avec un dérivé d'acide boronique de formule **(XIV) :** 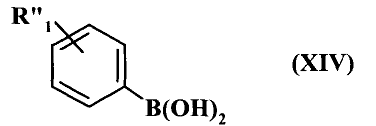 dans laquelle R"₁ représente un groupement cyano ou un hétérocycle,
      pour conduire, (après transformation éventuelle du groupement R"₁ quand celui-ci représente un groupement cyano en groupement tétrazolyle correspondant), au composé de formule **(I/a**_{**1**}**)** ou **(I/a**_{**2**}**),** cas particuliers des composés de formule (1) : 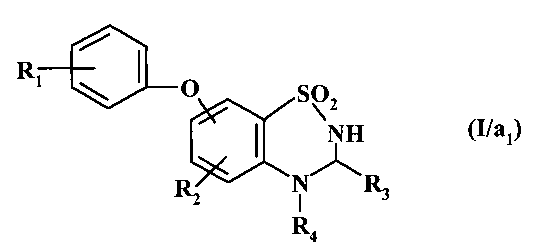 dans laquelle R₁, R₂, R₃ et R₄ ont la même signification que dans la formule (I), 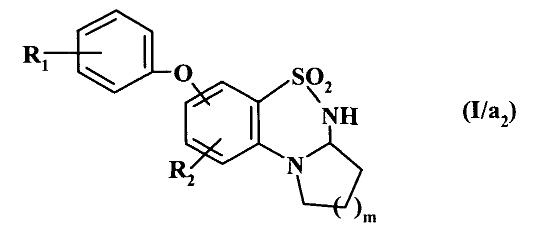 dans laquelle R₁, R₂ et m ont la même signification que dans la formule (I),
      composés de formule (I/a₁) ou (I/a₂) :
      que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

Le procédé de préparation des composés de formule (I) dans laquelle A représente un groupement CR₄R₅ est caractérisé en ce que l'on utilise comme produit de départ, un composé de formule **(XV)** : 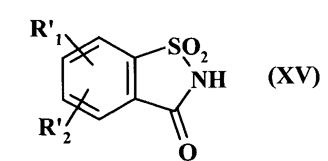 dans laquelle :
R'₁ représente un groupement alkoxy (C₁-C₆) linéaire ou ramifié,
R'₂ représente un atome d'hydrogène, d'halogène ou un groupement alkoxy (C₁-C₆) linéaire
ou ramifié,
qui subit l'action de la chloroacétone en présence de diméthylformamide, pour conduire au composé de formule **(XVI) :** 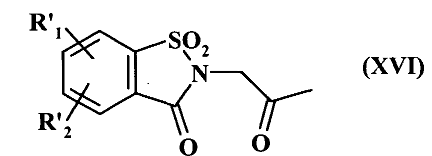 dans laquelle R'₁ et R'₂ ont la même signification que précédemment,
qui subit un réarrangement en milieu basique, pour conduire au composé de formule **(XVII) :** 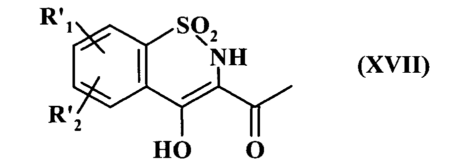 dans laquelle R'₁ et R'₂ ont la même signification que précédemment,
qui est déacétylé par chauffage au reflux en milieu benzénique en présence d'un excès d'éthylèneglycol et d'une quantité catalytique d'acide *p*-toluène sulfonique, pour conduire au composé de formule **(XVIII) :** 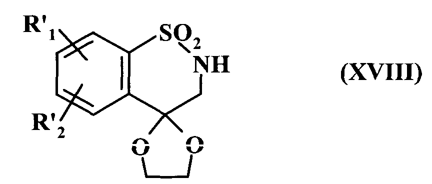 dans laquelle R'₁ et R'₂ ont la même signification que précédemment,
qui subit une hydrolyse en milieu acide, pour conduire au composé de formule **(XIXa) :** 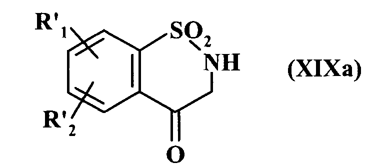 dans laquelle R'₁ et R'₂ ont la même signification que précédemment,
dont, éventuellement, selon la nature du groupement R₃ que l'on souhaite obtenir, on protège l'atome d'azote par un groupement protecteur, puis, après traitement par une base forte, que l'on traite par un composé de formule R'₃-P,
dans laquelle R'₃ représente un groupement alkyle (C₁-C₆) linéaire ou ramifié ou un groupement cycloalkyle (C₃-C₇) et P représente un groupe partant,
pour conduire après déprotection de l'atome d'azote, au composé de formule **(XIX'a) :** 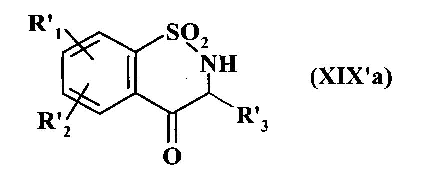 dans laquelle R'₁, R'₂ et R'₃ ont la même signification que précédemment,
composé de formule (XIXa) et (XIX'a) représentés par la formule **(XIX)** : 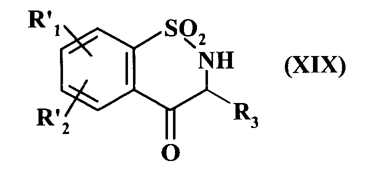 dans laquelle R'₁ et R'₂ ont la même signification que précédemment et R₃ est tel que défini dans la formule (I),
qui :
- **soit** subit une réduction catalytique, pour conduire au composé de formule **(XX)** : 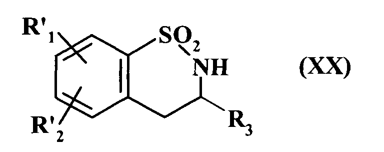 dans laquelle R'₁ et R'₂ ont la même signification que précédemment,
- **soit** est transformé par l'action d'un hydrure en alcool dont on transforme le groupement hydroxy en atome d'halogène par l'action d'un réactif approprié,
   pour conduire au composé de formule **(XXI) :** 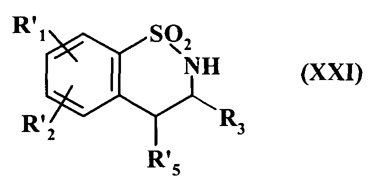 dans laquelle R'₁, R'₂ et R₃ ont la même signification que précédemment, et R'₅ représente un atome d'halogène ,
- **soit** subit l'action d'un organomagnésien R'₄MgBr dans lequel R'₄ représente un groupement alkyle (C₁-C₆) linéaire ou ramifié,
   pour conduire au composé de formule **(XIXb) :** 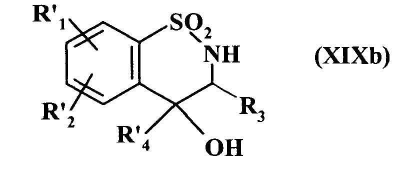 dans laquelle R'₁, R'₂, R₃ et R'₄ ont la même signification que précédemment,
   composé de formule **(XIXb) :**
- **ou bien** qui subit une réduction catalytique, pour conduire au composé de formule **(XXII) :** 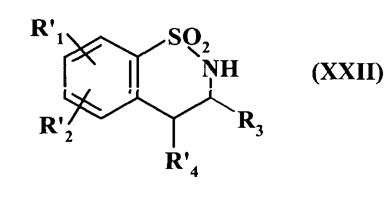 dans laquelle R'₁, R'₂, R₃ et R'₄ ont la même signification que précédemment,
- **ou bien** dont on transforme le groupement hydroxy en atome d'halogène par l'action d'un réactif approprié,
   pour conduire au composé de formule **(XXIII) :** 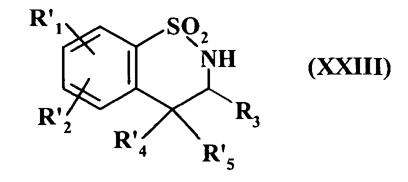
dans laquelle R'₁, R'₂, R₃ et R'₄ ont la même signification que précédemment et R'₅ représente un atome d'halogène,
composé de formule **(XX)** à **(XXIII)** dont on transforme le groupement R'₁ et le groupement R'₂ lorsque celui-ci représente un groupement alkoxy (C₁-C₆) linéaire ou ramifié, en groupements hydroxy,
pour conduire au composé de formule **(XXIV)** : 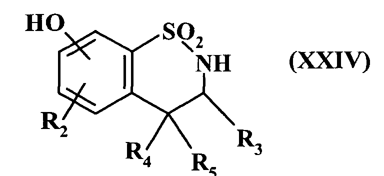 dans laquelle R₂, R₃, R₄ et R₅ ont la même signification que dans la formule (I),
composé de formule **(XXIV),**
que l'on fait réagir avec un dérivé d'acide boronique de formule **(XIV)** : 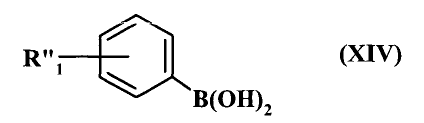 dans laquelle R"₁ représente un groupement cyano ou un hétérocycle,
pour conduire, (après transformation éventuelle du groupement R"₁ quand celui-ci représente un groupement cyano en groupement tétrazolyle correspondant), au composé de formule **(I/b),** cas particuliers des composés de formule (I) : 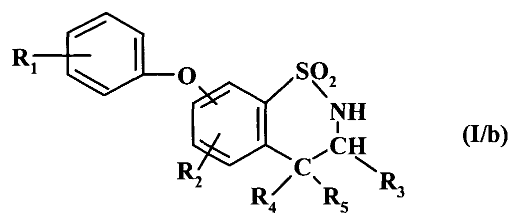 dans laquelle R₁, R₂, R₃, R₄ et R₅ ont la même signification que dans la formule (I),
composé de formule (I/b), que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention mais ne la limitent en aucune façon.

Les produits de départ utilisés sont des produits connus ou préparés selon des modes opératoires connus.

Les structures des composés décrits dans les exemples ont été déterminées selon les techniques spectrophotométriques usuelles (infrarouge, RMN, spectrométrie de masse ...).

### EXEMPLE 1 : 7-[3-(1H-Tétrazol-5-yl)phénoxy]-2,3,3a,4-tétrahydro-1H-pyrrolo [2,1-c] [1,2,4]benzothiadiazine-5,5-dioxide

### Stade A : 3-[(5,5-Dioxido-2,3-dihydro-1H-pyrrolo[2,1-c][1,2,4]benzothiadiazin-7-yl) oxy]phénol

A une solution de 27,50 mmoles 7-méthoxy-2,3-dihydro-1*H*-pyrrolo[2,1-*c*][1,2,4] benzothiadiazine 5,5-dioxide dans 350 ml de chlorure de méthylène refroidie à 0°C, on additionne goutte à goutte une solution 68,75 mmoles de BBr₃ dans 25 ml de chlorure de méthylène. On poursuit l'agitation 24 h à température ambiante. La réaction est versée dans un mélange glace/eau et la suspension est agitée 30 min. Le précipité est filtré, rincé plusieurs fois avec de l'eau, essoré et séché sous vide pour donner le produit attendu.

### Point de fusion : > 300 °C

| *Micro-analyse élémentaire :* | | | | |
|---|---|---|---|---|
| | *C%* | *H%* | *N%* | S% |
| *Calculée* | *50,41* | *4,23* | *11,76* | *13,46* |
| *Trouvée* | *50,00* | *4,19* | *11,28* | *13,41* |

### Stade B : 3-[(5,5-Dioxido-2,3-dihydro-1H-pyrrolo[2,1-c][1,2,4]benzothiadiazin-7-yl) oxy]benzonitrile

Dans 200 mi de chlorure de méthylène on agite pendant 24 h une suspension contenant 7,06 mmoles du produit décrit au stade précédent, 11,02 mmoles d'acide 3-cyanophényl boronique, 11,02 mmoles d'acétate de cuivre (II), 22 mmoles de pyridine et d'environ 500 mg de tamis moléculaire 4 Å. Le milieu réactionnel est dilué en ajoutant 100 ml supplémentaires de chlorure de méthylène et on filtre la suspension. Le filtrat est concentré puis directement déposé sur une colonne de silice que l'on élue avec un système 95/5 chlorure de méthylène/méthanol. Les fractions contenant le produit attendu sont rassemblés, évaporés et le résidu est repris dans un peu d'éther éthylique. Après filtration du solide on récupère le produit attendu sous forme de poudre blanche.

### Point de fusion : 229-233 °C

| *Micro-analyse élémentaire :* | | | | |
|---|---|---|---|---|
| | *C%* | *H%* | *N%* | S% |
| *Calculée* | *60,17* | *3,86* | *12,38* | *9,45* |
| *Trouvée* | *59,42* | *3,96* | *12,29* | *9,63* |

### Stade C : 7-[3-(1H-Tétrazol-5-yl)phénoxy]-2,3-dihydro-1H-pyrrolo[2,1-c][1,2,4] benzothiadiazine 5,5-dioxide

A une solution contenant 3,53 mmoles du produit obtenu au stade précédent et 7,07 mmoles d'azidotriméthylsilane dans 50 ml de dioxane, on ajoute 0,35 mmoles d'oxyde de di-(nbutyl)-étain et on chauffe le mélange 24 h à reflux. On laisse revenir à température ambiante, évapore le dioxane, reprend le résidu en solution dans le méthanol, concentre à nouveau. Le résidu est repris dans l'acétate d'éthyle et on extrait avec une solution de bicarbonate de sodium 10%. Les phases aqueuses basiques sont rassemblées, lavées un fois avec de l'acétate d'éthyle et acidifiées avec HCl 1N. Le précipité blanc formé est filtré pour donner le produit attendu.

### Point de fusion : 155 °C

| *Micro-analyse élémentaire :* | | | | |
|---|---|---|---|---|
| | *C%* | *H%* | *N%* | S% |
| *Calculée* | *53,40* | *3,69* | *21,98* | *8,39* |
| *Trouvée* | *53,17* | *3,67* | *22,19* | *7,81* |

### Stade D: 7-[3-(1H-Tétrazol-5-yl)phénoxy]-2,3,3a,4-tétrahydro-1H-pyrrolo[2,1-c] [1,2,4]benzothiadiazine 5,5-dioxide

On porte au reflux de 10 ml d'éthanol, 0,52 mmoles du produit obtenu au stade précédent en présence de 1,58 mmoles de NaBH₄ pendant 30 min. On laisse revenir à température ambiante et ajoute 10 ml d'HCl 1N. La suspension est agitée puis filtrée pour donner le produit attendu sous forme de poudre blanche. Celui ci est recristallisé dans l'éthanol et conduit au produit du titre.

### Point de fusion : 266-272 °C

| *Micro-analyse élémentaire :* | | | | |
|---|---|---|---|---|
| | *C%* | *H%* | *N%* | S% |
| *Calculée* | *53,12* | *4,20* | *21,86* | *8,34* |
| *Trouvée* | *53,14* | *4,36* | *21,93* | *8,56* |

### EXEMPLE 2 : 7-[3-(1-Méthyl-1H-tétrazol-5-yl)phénoxy]-2,3,3a,4-tétrahydro-1H-pyrrolo[2,1-c] [1,2,4]benzothiadiazine-5,5-dioxide

### Stade A : 7-[3-(1-Méthyl-2H-tétrazol-5-yl)phénoxy]-2,3-dihydro-1H-pyrrolo[2,1-c] [1,2,4]benzothiadiazine 5,5-dioxide

A une suspension du produit obtenu dans l'exemple 1 (300 mg, 0,78 mmol) dans 20 ml d'éthanol anhydre on ajoute 200 µl (0,39 mmol) d'oxyde de bis(tri-*n*-butyl) étain et porte au reflux la solution pendant 10 min. On évapore l'éthanol, ajoute 1 ml de iodure de méthyle et agite une nuit à température ambiante. Le précipité est filtré pour donner un mélange des isomères 1- et 2-méthyl-2*H*-tétrazol qui est purifié pour conduire au produit du titre.

### Stade B : 7-[3-(1-Méthyl-2H-tétrazol-5-yl)phénoxy]-2,3,3a,4-tetrahydro-1H-pyrrolo[2,1-c][1,2,4]benzothiadiazine 5,5-dioxide

On procède comme dans le stade D de l'exemple 1 en partant du produit obtenu au stade A.

### Point de fusion : 202-204 °C

| *Micro-analyse élémentaire :* | | | | |
|---|---|---|---|---|
| | *C%* | *H%* | *N%* | *S%* |
| *Calculée* | *54,26* | *4,55* | *21,09* | *8,05* |
| *Trouvée* | *54,14* | *4,63* | *20,30* | *7,88* |

### EXEMPLE 3 : 7-[3-(2-Méthyl-2H-tétrazol-5-yl)phénoxy]-2,3,3a,4-tétrahydro-1H-pyrrolo[2,1-c] [1,2,4]benzothiadiazine-5,5-dioxide

### Stade A : 7-[3-(2-Méthyl-2H-tétrazol-5-yl)phénoxy]-2,3-dihydro-1H-pyrrolo[2,1-c] [1,2,4]benzothiadiazine 5,5-dioxide

A une solution du produit obtenu au stade C de l'exemple 1 (500 mg) dans 4 ml d'eau et 105 mg de NaOH on ajoute goutte à goutte 163 µl de iodure de méthyle diluée dans 4 ml d'acétonitrile. On porte à reflux 2h et laisse la réaction revenir à température ambiante. Il se forme un précipité que l'on filtre et qui correspond au produit du titre.

| *Micro-analyse élémentaire :* | | | | |
|---|---|---|---|---|
| | *C%* | *H%* | *N%* | *S%* |
| *Calculée* | *54,54* | *4,07* | *21,20* | *8,09* |
| *Trouvée* | *54,52* | *4,24* | *20,72* | *8,18* |

### Stade B : 7-[3-(2-Méthyl-2H-tétrazol-5-yl)phénoxy]-2,3,3a,4-tétrahydro-1H-pyrrolo[2,1-c][1,2,4]benzothiadiazine 5,5-dioxide

On procède comme dans le stade D de l'exemple 1 à partir du composé obtenu au stade A.

### Point de fusion : 266 °C

| *Micro-analyse élémentaire :* | | | | |
|---|---|---|---|---|
| | *C%* | *H%* | *N%* | *S%* |
| *Calculée* | *54,26* | *4,55* | *21,09* | *8,05* |
| *Trouvée* | *54,06* | *4,78* | *20,01* | *8,19* |

Les exemples 4 à 12 suivants ont été préparés selon le procédé décrit dans l'exemple 1 en utilisant au stade B l'acide boronique correspondant et en transformant le groupement fonctionnel en hétéroaryle souhaité.

### EXEMPLE 4 : 7-[3-(1H-Imidazol-2-yl)phénoxy]-2,3,3a,4-tétrahydro-1H-pyrrolo [2,1-c] [1,2,4]benzothiadiazine-5,5-dioxide

### EXEMPLE 5 : 7-[3-(1H-Imidazol-1-yl)phénoxy]-2,3,3a,4-tétrahydro-1H-pyrrolo [2,1-c][1,2,4]benzothiadiazine-5,5-dioxide

### EXEMPLE 6 : 7-[3-(1H-Imidazol-5-yl)phénoxy]-2,3,3a,4-tétrahydro-1H-pyrrolo [2,1-c][1,2,4]benzothiadiazine-5,5-dioxide

### EXEMPLE 7 : 7-[3-(1H-1,2,3-Triazol-5-yl)phénoxy]-2,3,3a,4-tétrahydro-1H-pyrrolo [2,1-c] [1,2,4]benzothiadiazine-5,5-dioxide

### EXEMPLE 8 : 7-[3-(2H-1,2,3-Triazol-2-yl)phénoxy]-2,3,3a,4-tétrahydro-1H-pyrrolo [2,1-c][1,2,4]benzothiadiazine-5,5-dioxide

### EXEMPLE 9 : 7-[3-(1H-1,2,3-Triazol-1-yl)phénoxy]-2,3,3a,4-tétrahydro-1H-pyrrolo [2,1-c][1,2,4]benzothiadiazine-5,5-dioxide

### EXEMPLE 10 : 7-[3-(4H-1,2,4-Triazol-3-yl)phénoxy]-2,3,3a,4-tétrahydro-1H-pyrrolo [2,1-c] [1,2,4]benzothiadiazine-5,5-dioxide

### EXEMPLE 11 : 7-[3-(1H-1,2,4-Triazol-1-yl)phénoxy]-2,3,3a,4-tétrahydro-1H-pyrrolo [2,1-c] [1,2,4]benzothiadiazine-5,5-dioxide

### EXEMPLE 12 : 7-[3-(4H-1,2,4-Triazol-4-yl)phénoxy]-2,3,3a,4-tétrahydro-1H-pyrrolo [2,1-c][1,2,4]benzothiadiazine-5,5-dioxide

### EXEMPLE 13 : 3-{3-[(5,5-Dioxido-2,3,3a,4-tétrahydro-1H-pyrrolo[2,1-c][1,2,4] benzothiadiazin-7-yl)oxy]phényl}-1,2,4-oxadiazol-5(4H)-one

### Stade A : 3-[(5,5-Dioxido-2,3-dihydro-1H-pyrrolo[2,1-c][1,2,4]benzothiadiazin-7-yl)oxy]-N'-hydroxybenzènecarboximidamide

A une solution du chlorhydrate d'hydroxylamine (307 mg, 4,42 mmol) dans 1,5 ml de DMSO on ajoute 616 µl (4,42 mmol) de triéthylamine et agite la suspension 10 minutes. Le précipité est filtré et le filtrat est concentré. A ce filtrat, on ajoute ensuite 250 mg (0,737 mmol) du produit obtenu au stade B de l'exemple 1 et on agite la solution à 75°C pendant 16h. On laisse revenir à température ambiante et précipite le milieu réactionnel dans l'eau. On observe une pâte gommeuse blanche que l'on durcit par addition de CH₂Cl₂. Le précipité est filtré pour conduire au produit du titre.

### Point de fusion : 165-169°C

| *Micro-analyse élémentaire :* | | | | |
|---|---|---|---|---|
| | *C%* | *H%* | *N%* | *S%* |
| *Calculée* | *54,83* | *4,33* | *15,04* | *8,61* |
| *Trouvée* | *55,07* | *4,18* | *14,66* | *8,95* |

### Stade B : 3-[(5,5-Dioxido-2,3-dihydro-1H-pyrrolo[2,1-c][1,2,4]benzothiadiazin-7-yl) oxy]-N'-[(éthoxycarbonyl)oxy]benzènecarboximidamide

A une solution refroidie à 0°C du produit obtenu au stade A (244 mg, 0,65 mmol) dans 1,5 ml de DMF et 59 µl (0,72 mmol) de pyridine on ajoute goutte à goutte 65 µl (0,65 mmol) de chloroformiate d'éthyle. On poursuit l'agitation à 0°C pendant 30 min et précipite le milieu réactionnel dans l'eau. Le précipité est filtré pour conduire au produit du titre.

### Point de fusion : 101-105 °C

### Stade C : 3-{3-[(5,5-Dioxido-2,3,-dihydro-1H-pyrrolo[2,1-c][1,2,4]benzothiadiazin-7-yl)oxy]phényl}-1,2,4-oxadiazol-5(4H)-one

Le produit obtenu au stade précédent (220 mg, 0,49 mmol) est agité au reflux du xylène (3 ml) pendant 2 h. On laisse revenir à température ambiante et filtre le précipité pour conduire au produit du titre.

### Point de fusion : > 250°C

### Stade D : 3-{3-[(5,5-Dioxido-2,3,3a,4-tétrahydro-1H-pyrrolo[2,1-c][1,2,4] benzothiadiazin-7-yl)oxy]phényl} -1,2,4-oxadiazol-5(4H)-one

On procède comme dans le stade D de l'exemple 1 en partant du produit obtenu au stade C.

### Point de fusion : 241-244°C

| *Micro-analyse élémentaire :* | | | | |
|---|---|---|---|---|
| | *C%* | *H%* | *N%* | *S%* |
| *Calculée* | *53,99* | *4,03* | *13,99* | *8,01* |
| *Trouvée* | *54,28* | *4,01* | *13,50* | *7,92* |

### EXEMPLE 14 : 3-{3-[(5,5-Dioxido-2,3,3a,4-tétrahydro-1H-pyrrolo[2,1-c][1,2,4] benzothiadiazin-7-yl)oxy]phényl)-1,2,4-oxadiazole-5(4H)-thione

### Stade A : 3-{3-[(5,5-Dioxido-2,3-dihydro-1H-pyrrolo[2,1-c][1,2,4]benzothiadiazin-7-yl)oxy]phényl}-1,2,4-oxadiazole-5(4H)-thione

A une suspension du produit obtenu au stade A de l'exemple 13 (300 mg, 0,81 mmol) dans 6 ml de CH₃CN, on ajoute 239 mg (1,21 mmol) de thiocarbonyldiimidazole puis 481 µl (3,22 mmol) de DBU. La solution réactionnelle est agitée 1 h à température ambiante. On évapore à sec et chromatographie sur silice (95/5 CH₂Cl₂/MeOH). Après évaporation des fractions contenant le produit formé le résidu est trituré dans du MeOH et le produit du titre est récupéré par filtration.

### Point de fusion : 228-230°C

| *Micro-analyse élémentaire :* | | | | |
|---|---|---|---|---|
| | *C%* | *H%* | *N%* | S% |
| *Calculée* | *52,16* | *3,40* | *13,52* | *15,47* |
| *Trouvée* | *54,27* | *3,72* | *13,43* | *15,76* |

### Stade B : 3-{3-[(5,5-Dioxido-2,3,3a,4-tétrahydro-1H-pyrrolo[2,1-c][1,2,4] benzothiadiazin-7-yl)oxy]phényl} -1,2,4-oxadiazole-5(4H)-thione

On procède comme dans le stade D de l'exemple 1 en partant du produit obtenu au stade A.

### Point de fusion : 231-235°C

| *Micro-analyse élémentaire :* | | | | |
|---|---|---|---|---|
| | *C%* | *H%* | *N%* | *S%* |
| *Calculée* | *51,91* | *3, 87* | *13,45* | *15,40* |
| *Trouvée* | *51,68* | *3,98* | *13,07* | *15,49* |

### EXEMPLE 15 : 7-[3-(2-Oxido-3H-1,2,3,5-oxathiadiazol-4-yl)phénoxy]-2,3,3a,4-tétrahydro-1H-pyrrolo[2,1-c][1,2,4]benzothiadiazine 5,5-dioxide

### Stade A : 7-[3-(2-Oxido-3H-1,2,3,5-oxathiadiazol-4-yl)phénoxy]-2,3-dihydro-1H-pyrrolo[2,1-c][1,2,4]benzothiadiazine 5,5-dioxide

A une suspension refroidie à 0°C du produit obtenu au stade A de l'exemple 13 (250 mg, 0,67 mmol) dans 15 ml de THF on ajoute 109 µl (1,34 mmol) de pyridine puis goutte à goutte 72 µl (1,01 mmol) de chlorure de thionyle en solution dans 3 ml de CH₂Cl₂. On poursuit l'agitation à 0°C pendant 30 min puis laisse remonter à température ambiante en agitant 1 h supplémentaire. On évapore à sec et concrétise le résidu en le triturant dans de l'eau. Le précipité est filtré pour conduire au produit du titre.

### Stade B : 7-[3-(2-Oxido-3H-1,2,3,5-oxathiadiazol-4-yl)phénoxy]-2,3,3a,4-tétrahydro-1H-pyrrolo[2,1-c][1,2,4]benzothiadiazine 5,5-dioxide

On procède comme au stade D de l'exemple 1 en partant du produit obtenu au stade A.

### Point de fusion : 178-182°C

| *Micro-analyse élémentaire :* | | | | |
|---|---|---|---|---|
| | *C%* | *H%* | *N%* | *S%* |
| *Calculée* | *48,56* | *3,84* | *13,32* | *15,25* |
| *Trouvée* | *48,62* | *3,84* | *13,27* | *15,49* |

### EXEMPLE 16 : 7-[3-(2-Furyl)phénoxy]-2,3,3a,4-tétrahydro-1H-pyrrolo[2,1-c][1,2,4] benzothiadiazine 5,5-dioxide

### Stade A : 7-(3-Bromophénoxy)-2,3-dihydro-1H-pyrrolo[2,1-c][1,2,4]benzothiadiazine 5,5-dioxide

On procède comme au stade B de l'exemple 1 en utilisant comme produit de départ l'acide (3-bromophényl)boronique et le produit obtenu au stade A de l'exemple 1.

### Stade B : 7-[3-(2-Furyl)phénoxy]-2,3,-dihydro-1H-pyrrolo[2,1-c][1,2,4]benzo thiadiazine 5,5-dioxide

On chauffe au reflux pendant 45 min une suspension du produit obtenu au stade A (200 mg, 0,50 mmol), 222 µl (0,66 mmol) de 2-(tributylstannyl)furane et 30 mg (0,025 mmol) de tétrakistriphénylphosphine dans 4 ml de toluène. Le toluène est évaporé sous vide et le résidu est chromatographié sur SiO₂ (CH₂Cl₂/acétone 96/4) pour conduire au produit du titre.

### Point de fusion : 186-189°C

| *Micro-analyse élémentaire :* | | | | |
|---|---|---|---|---|
| | *C%* | *H%* | *N%* | *S%* |
| *Calculée* | *63,15* | *4,24* | *7,36* | *8,43* |
| *Trouvée* | *62,85* | *4,22* | *7,37* | *8,40* |

### Stade C : 7-[3-(2-Furyl)phénoxy]-2,3,3a,4-tétrahydro-1H-pyrrolo[2,1-c][1,2,4]benzo thiadiazine 5,5-dioxide

On procède comme au stade D de l'exemple 1 en partant du produit obtenu au stade B.

### Point de fusion : 178-180°C

| *Micro-analyse élémentaire :* | | | | |
|---|---|---|---|---|
| | *C%* | *H%* | *N%* | *S%* |
| *Calculée* | *62,81* | *4,74* | *7,32* | *8,38* |
| *Trouvée* | *63,11* | *4,82* | *7,28* | *8,34* |

### EXEMPLE 17 : 7-[3-(3-Furyl)phénoxy]-2,3,3a,4-tétrahydro-1H-pyrrolo[2,1-c][1,2,4] benzothiadiazine 5,5-dioxide

### Stade A : 7-[3-(3-Furyl)phénoxy]-2,3-dihydro-1H-pyrrolo[2,1-c][1,2,4] benzothiadiazine 5,5-dioxide

On chauffe à 80°C pendant 3h sous N₂ une suspension du produit obtenu au stade A de l'exemple 16 (300 mg, 0,76 mmol), 111 mg (0,99 mmol) d'acide 3-furaneboronique, 137 mg (0,99 mmol) de K₂CO₃ et 45 mg (0,038 mmol) de tétrakistriphénylphosphine dans un mélange de 6 ml d'éthanol et 1,8 ml d'eau. On évapore sous vide l'éthanol et reprend dans de l'eau et HCl 1N. Le précipité est filtré pour conduire au produit du titre.

### Point de fusion : 128-132°C

### Stade B : 7-[3-(3-Furyl)phénoxy]-2,3,3a,4-tétrahydro-1H-pyrrolo[2,1-c][1,2,4] benzothiadiazine 5,5-dioxide

On procède comme au stade D de l'exemple 1 en partant du produit obtenu au stade A.

### Point de fusion : 186-189°C

| *Micro-analyse élémentaire :* | | | | |
|---|---|---|---|---|
| | *C%* | *H%* | *N%* | *S%* |
| *Calculée* | *62,81* | *4,74* | *7,32* | *8,38* |
| *Trouvée* | *62,43* | *4,77* | *7,33* | *8,43* |

### EXEMPLE 18 : 7-[3-(1,3-Oxazol-5-yl)phénoxy]-2,3,3a,4-tétrahydro-1H-pyrrolo[2,1-c] [1,2,4]benzothiadiazine 5,5-dioxide

On procède comme dans l'exemple 17 en remplaçant dans le stade A l'acide 3-furaneboronique par l'acide 1,3-oxazol-5-ylboronique.

### Point de fusion : 210°C

| *Micro-analyse élémentaire :* | | | | |
|---|---|---|---|---|
| | C% | *H%* | *N%* | *S%* |
| *Calculée* | *59,52* | *4,47* | *10,96* | *8,36* |
| *Trouvée* | *59,47* | *4,51* | *10,68* | *8,74* |

### EXEMPLE 19 : 7-(3-Pyridin-2-ylphénoxy)-2,3,3a,4-tétrahydro-1H-pyrrolo[2,1-c] [1,2,4]benzothiadiazine 5,5-dioxide

### Stade A : 7-(3-Pyridin-2-ylphénoxy)-2,3-dihydro-1H-pyrrolo[2,1-c][1,2,4] benzothiadiazine 5,5-dioxide

On procède comme au stade B de l'exemple 16 en utilisant la 2-(tributylstannyl)pyridine comme produit de départ.

### Point de fusion : 205-207°C

### Stade B : 7-(3-Pyridin-2-ylphénoxy)-2,3,3a,4-tétrahydro-1H-pyrrolo[2,1-c][1,2,4] benzothiadiazine 5,5-dioxide

On procède comme au stade D de l'exemple 1 en partant du produit obtenu au stade A.

### Point de fusion : 129°C

| *Micro-analyse élémentaire :* | | | | |
|---|---|---|---|---|
| | *C%* | *H%* | *N%* | *S%* |
| *Calculée* | *64,10* | *4,87* | *10,68* | *8,15* |
| *Trouvée* | *64,30* | *5,07* | *10,55* | *7,71* |

### EXEMPLE 20 : 7-[3-(1H-Pyrrol-1-yl)phénoxy]-2,3,3a,4-tétrahydro-1H-pyrrolo [2,1-c][1,2,4]benzothiadiazine 5,5-dioxide

Dans un mélange biphasique de 2,5 ml d'eau, 1,25 ml d'AcOH, 3,75 ml de dichlorol,2-éthane on ajoute 250 mg (0,75 mmol) de {3-[(5,5-dioxido-2,3,3a,4-tétrahydro-1*H*-pyrrolo[2,1-*c*][1,2,4]benzothiadiazin-7-yl)oxy]phényl}amine et 137 µl (1,06 mmol) de 2,5 dimethoxytétrahydrofurane. On agite 1 h à 80°C, laisse revenir à température ambiante et extrait avec CH₂Cl₂. La phase organique est lavée par une solution aqueuse saturée de NaCl, séchée sur MgSO₄. Le produit du titre est purifié par chromatographie sur colonne de silice (CH₂Cl₂/heptane 70/30).

### Point de fusion : 169-170 °C

| *Micro-analyse élémentaire :* | | | | |
|---|---|---|---|---|
| | *C%* | *H%* | *N%* | *S%* |
| *Calculée* | *62,97* | *5,02* | *11,02* | *8,41* |
| *Trouvée* | *63,14* | *5,18* | *10,85* | *8,15* |

### EXEMPLE 21 : 7-(3-Morpholin-4-ylphénoxy)-2,3,3a,4-tétrahydro-1H-pyrrolo[2,1-c] [1,2,4]benzothiadiazine 5,5-dioxide

On chauffe une nuit au reflux un mélange de 300 mg (0,90 mmol) de {3-[(5,5-dioxido-2,3,3a,4-tétrahydro-1*H*-pyrrolo[2,1-*c*][1,2,4]benzothiadiazin-7-yl)oxy]phényl}amine, 192 mg de Na₂CO₃ (1,81 mmol), 5 mg de NaI, 126 µl de 2-bromoéthyléther (0,905 mmol) dans 6 ml de CH₃CN. On filtre la suspension, évapore le filtrat, le reprend dans CH₂Cl₂, le lave avec une solution saturée en NaCl. Après évaporation sous vide on purifie le produit attendu par chromatographie sur silice (CH₂Cl₂/acétone 96/4).

### Point de fusion : 199-202°C

| *Micro-analyse élémentaire :* | | | | |
|---|---|---|---|---|
| | *C%* | *H%* | *N%* | *S%* |
| *Calculée* | *59,83* | *5,77* | *10,47* | *7,99* |
| *Trouvée* | *59,38* | *6,05* | *10,31* | *8,03* |

### EXEMPLE 22 : 7-[4-(1H-Tétrazol-5-yl)phénoxy]-2,3,3a,4-tétrahydro-1H-pyrrolo [2,1-c][1,2,4]benzothiadiazine 5,5-dioxide

### Stade A : 4-[(5,5-Dioxido-2,3-dihydro-1H-pyrrolo[2,1-c][1,2,4]benzothiadiazin-7-yl) oxy]benzonitrile

On procède comme au stade B de l'exemple 1 en remplaçant l'acide 3-cyanophényl-boronique par l'acide 4-cyanophénylboronique.

### Point de fusion : 239-242°C

### Stade B : 7-[4-(1H-Tétrazol-5-yl)phénoxy]-2,3-dihydro-1H-pyrrolo [2,1-c][1,2,4] benzothiadiazine 5,5-dioxide

On procède comme au stade C de l'exemple 1 en utilisant comme produit de départ le produit obtenu au stade A.

### Stade C : 7-[4-(1H-Tétrazol-5-yl)phénoxy]-2,3,3a,4-tétrahydro-1H-pyrrolo [2,1-c] [1,2,4]benzothiadiazine 5,5-dioxide

On procède comme au stade D de l'exemple 1 en utilisant comme produit de départ le produit obtenu au stade B.

### Point de fusion : 215-219°C

| *Micro-analyse élémentaire :* | | | | |
|---|---|---|---|---|
| | *C%* | *H%* | *N%* | S% |
| *Calculée* | *53,12* | *4,20* | *21,86* | *8,34* |
| *Trouvée* | *52,98* | *4,11* | *21,32* | *8,68* |

### EXEMPLE 23 : 5-{3-[(5,5-Dioxido-2,3,3a,4-tétrahydro-1H-pyrrolo[2,1-c][1,2,4] benzothiadiazin-7-yl)oxy]phényl}-1,3,4-oxadiazol-2(3H)-one

### Stade A : 3-[(5,5-Dioxido-2,3-dihydro-1H-pyrrolo[2,1-c][1,2,4] benzothiadiazin-7-yl)oxy]benzoate de méthyle

On procède comme au stade B de l'exemple 1 en remplaçant l'acide 3-cyanophényl-boronique par l'acide [3-(méthoxycarbonyl)phényl]boronique.

### Point de fusion : 239-242°C

| *Micro-analyse élémentaire :* | | | | |
|---|---|---|---|---|
| | *C%* | *H%* | *N%* | *S%* |
| *Calculée* | *58,06* | 4,33 | *7,52* | 8,61 |
| *Trouvée* | *52,53* | *4,01* | *7,84* | *9,00* |

### Stade B : 3-[(5,5-Dioxido-2,3-dihydro-1H-pyrrolo[2,1-c][1,2,4]benzothiadiazin-7-yl) oxy]benzohydrazide

On chauffe à reflux pendant 24 h une suspension de 500 mg (1,34 mmol) du produit obtenu au stade A et 978 µl (20,13 mmol) de monohydrate d'hydrazine dans un mélange MeOH (30 ml) et DMF (6 ml). Le MeOH est évaporé, on rajoute de l'eau au milieu réactionnel et filtre le précipité qui correspond au produit du titre .

### Point de fusion : 197-201 °C

### Stade C : 5-{3-[(5,5-Dioxido-2,3-dihydro-1H-pyrrolo[2,1-c][1,2,4] benzothiadiazin-7-yl)oxy]phényl}-1,3,4-oxadiazol-2(3H)-one

On chauffe à reflux pendant 3 h une suspension de 150 mg (0,40 mmol) du produit obtenu au stade B, 85 mg (0,52 mmol) de 1,1'-carbonyldiimidazole et 95 µl (0,68 mmol) de Et₃N dans un mélange THF (3,7 ml) et DMF (3,7 ml). On laisse revenir à température ambiante et acidifie avec HCl 1N. Il se forme un précipité blanc qui est filtré pour conduire au produit du titre.

### Stade D : 5-{3-[(5,5-Dioxido-2,3,3a,4-tétrahydro-1H-pyrrolo[2,1-c][1,2,4] benzothiadiazin-7-yl)oxy]phényl}-1,3,4-oxadiazol-2(3H)-one

On procède comme au stade D de l'exemple 1 en partant du produit obtenu au stade C.

### Point de fusion : 254-258°C

| *Micro-analyse élémentaire :* | | | | |
|---|---|---|---|---|
| | *C%* | *H%* | *N%* | *S%* |
| *Calculée* | *53,99* | *4,03* | *13,99* | *8,01* |
| *Trouvée* | *54,31* | *4,27* | *13,24* | *7,72* |

### ETUDE PHARMACOLOGIQUE DES PRODUITS DE L'INVENTION

### Etude des courants excitateurs induit par l'AMPA dans les oocytes de Xenopus

### a - Méthode:

Des ARNm sont préparés à partir de cortex cérébral de rat mâle Wistar par la méthode au guanidium thiocyanate/phénol/chloroforme. Les ARNm Poly (A⁺) sont isolés par chromatographie sur oligo-dT cellulose et injectés à raison de 50 ng par oocyte. Les oocytes sont laissés 2 à 3 jours en incubation à 18°C pour permettre l'expression des récepteurs puis stockés à 8-10°C.

L'enregistrement électrophysiologique est réalisé dans une chambre en plexiglass® à 20-24°C en milieu OR2 (J. Exp. Zool., 1973, 184, 321-334) par la méthode du "voltage-clamp" à 2 électrodes, une 3ème électrode placée dans le bain servant de référence.

Tous les composés sont appliqués via le milieu d'incubation et le courant électrique est mesuré à la fin de la période d'application. L'AMPA est utilisé à la concentration de 10 µM. Pour chaque composé étudié, on définit la concentration doublant (EC2X) ou quintuplant (EC5X) l'intensité du courant induit par l'AMPA seul (5 à 50 nA).

### b - Résultats :

Les composés de l'invention potentialisent très fortement les effets excitateurs de LAMPA et leur activité est très nettement supérieure à celle des composés de référence. Le composé de l'exemple 1 possède notamment une EC2X égale à 0,2 µM et une EC5X égale à 0,8 µM, le composé de l'exemple 17 une EC2X égale à 4,5 µM et une EC5X égale à 11,5 µM, le composé de l'exemple 18 une EC2X égale à 0,66 µM et une EC5X égale à 4 µM.

### COMPOSITION PHARMACEUTIQUE

| | |
|---|---|
| Formule de préparation pour 1000 comprimés dosés à 100 mg de 7-[3-(3-Furyl) phénoxy]-2,3,3a,4-tétrahydro-1*H*-pyrrolo[2,1-*c*][1,2,4]benzothiadiazine 5,5-dioxide (Exemple 17) | 100 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 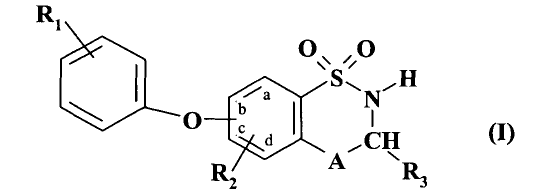 dans laquelle :
**R**_{**1**} représente un groupement hétérocyclique,
**R**_{**2**} représente un atome d'hydrogène, d'halogène ou un groupement hydroxy,
**A** représente un groupement CR₄R₅ ou un groupement NR₄,
**R**_{**3**} représente un atome d'hydrogène, un groupement alkyle (C₁-C₆) linéaire ou ramifié ou un groupement cycloalkyle (C₃-C₇),
**R**_{**4**} représente un atome d'hydrogène ou un groupement alkyle (C₁-C₆) linéaire ou ramifié,
ou bien
**A** représente un atome d'azote et forme avec le groupement -CHR₃- adjacent le cycle 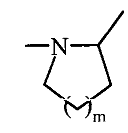 dans lequel m représente 1, 2 ou 3,
**R**_{**5**} représente un atome d'hydrogène ou d'halogène,
leurs énantiomères et diastéréoisomères ainsi que leurs sels d'addition à un acide ou à une base pharmaceutiquement acceptable,
étant entendu que par groupement hétérocyclique, on comprend groupement aromatique ou non, monocyclique ou bicyclique, contenant un à quatre hétéroatomes, identiques ou différents, choisis parmi azote, oxygène et soufre, éventuellement substitué par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, alkoxycarbonyle (C₁-C₆) linéaire ou ramifié, oxo, thioxo, carboxy, acyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié), ou alkyl (C₁-C₆) sulfonylamino.

2. Composés de formule (I) selon la revendication 1 tels que R₂ représente un atome d'hydrogène.

3. Composés de formule (I) selon l'une quelconque des revendication 1 ou 2 tels que le groupement 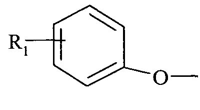 est position b du phényle qui le porte.

4. Composés de formule (I) selon l'une quelconque des revendications 1, 2 ou 3 tels que R₁ est un groupement hétérocyclique monocyclique.

5. Composés de formule (I) selon la revendication 4 tels que R₁ est un groupement hétérocyclique monocyclique aromatique.

6. Composés de formule (I) selon l'une quelconque des revendications 1 à 5 tels que R₁ est un groupement tétrazolyle, furanyle ou oxazolyle.

7. Composés de formule (I) selon l'une quelconque des revendications 1 à 6 tels que R₁ est en position méta du noyau phénoxy qui le porte.

8. Composés de formule (I) selon l'une quelconque des revendications 1 à 6 tels que R₁ est en position para du noyau phénoxy qui le porte.

9. Composés de formule (I) selon l'une quelconque des revendications 1 à 8 tels que A représente un atome d'azote et forme avec le groupement -CHR₃- adjacent le cyle 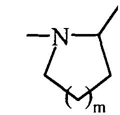 dans lequel m représente 1, 2 ou 3, et préférentiellement 1.

10. Composé de formule (I) qui est le 7-[3-(1*H*-tétrazol-5-yl)phénoxy]-2,3,3a,4-tétrahydro-1*H*-pyrrolo[2,1-*c*][1,2,4]benzothiadiazine-5,5-dioxide.

11. Composé de formule (I) qui est le 7-[3-(3-furyl)phénoxy]-2,3,3a,4-tétrahydro-1*H*-pyrrolo[2,1-*c*][1,2,4]benzothiadiazine 5,5-dioxide.

12. Composé de formule (I) qui est le 7-[3-(1,3-oxazol-5-yl)phénoxy]-2,3,3a,4-tétrahydro-1*H*-pyrrolo[2,1-*c*] [1,2,4]benzothiadiazine.

13. Procédé de préparation des composés de formule (I) selon la revendication 1 dans laquelle A représente un groupement NR₄ ou A représente un atome d'azote et forme avec le groupement CHR₃ adjacent le cycle 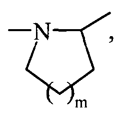 dans lequel m représente 1, 2 ou 3, **caractérisé en ce que** l'on utilise comme produit de départ un composé de formule **(II) :** 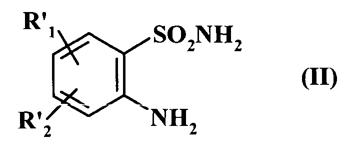 dans laquelle :
R'₁ représente un groupement alkoxy (C₁-C₆) linéaire ou ramifié,
R'₂ représente un atome d'hydrogène, d'halogène ou un groupement alkoxy (C₁-C₆) linéaire ou ramifié,
qui est :
**(a) soit mis en réaction** avec le chlorure d'acide de formule **(III)** en présence d'une base, en milieu tétrahydrofurane ou acétonitrile :
**Cl - CH**_{**2**} **- (CH**_{**2**}**)**_{**m**} **- CH**_{**2**} **- COCl (III)**
dans laquelle m a la même signification que dans la formule (I),
pour conduire au composé de formule **(IV) :** 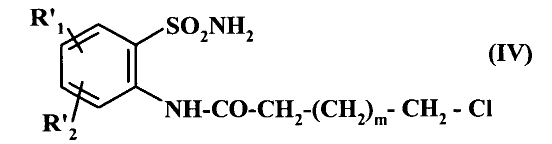 dans laquelle R'₁ et R'₂ sont tels que définis précédemment,
qui est alors cyclisé en milieu basique, pour conduire au composé de formule (V) 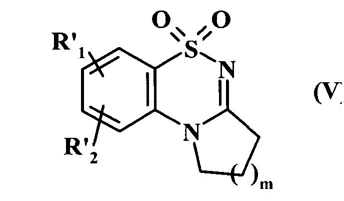 dans laquelle R'₁, R'₂ et m sont tels que définis précédemment,
qui subit éventuellement une réduction, en milieu alcoolique ou diméthylformamide, en présence de borohydrure de sodium, pour conduire au composé de formule **(VI) :** 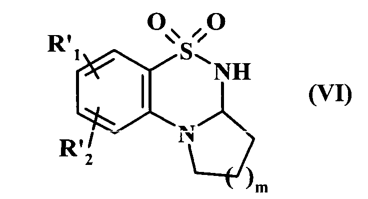 dans laquelle R'₁, R'₂ et m sont tels que définis précédemment,
composé de formule **(V)** ou **(VI)** qui subit l'action du tribromure de bore,
pour conduire au composé de formule **(VII)** : 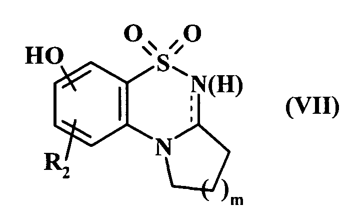 dans laquelle R₂ est tel que défini dans la formule (I) et m est tel que défini précédemment,
**(b) soit cyclisé :**
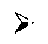 en présence d'une amidine de formule **(VIII)** : 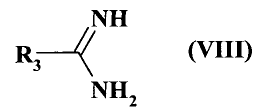 dans laquelle :
R₃ est tel que défini dans la formule (I),
pour conduire au composé de formule **(IX) :** 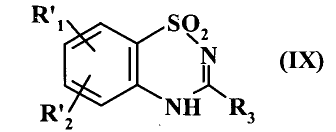 dans laquelle R'₁, R'₂, et R₃ ont la même signification que précédemment,
qui est :
◆ ou bien réduit par un hydrure métallique,
pour conduire au composé de formule **(X) :** 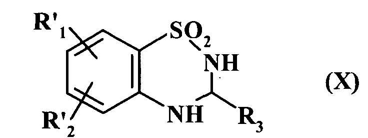 dans laquelle R'₁, R'₂, et R₃ ont la même signification que précédemment,
◆ ou bien alkylé par action d'une base forte en présence d'un agent d'alkylation R'₄X dans lequel R'₄ représente un groupement alkyle (C₁-C₆) linéaire ou ramifié et X un atome d'halogène, puis réduit,
pour conduire au composé de formule **(XI) :** 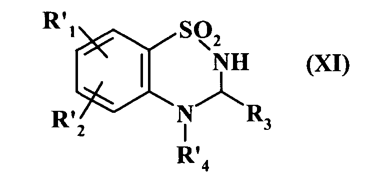
dans laquelle R'₁, R'₂, R₃ et R'₄ ont la même signification que précédemment,
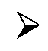 en présence d'un aldéhyde de formule **(XII) :** 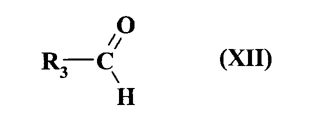 dans laquelle R₃ est tel que défini dans la formule (I),
pour conduire au composé de formule (X) décrit précédemment,
composé de formule (X) ou (XI),
dont on transforme le groupement R'₁ et le groupement R'₂ lorsque celui-ci représente un groupement alkoxy (C₁-C₆) linéaire ou ramifié, en groupements hydroxy,
pour conduire au composé de formule **(XIII) :** 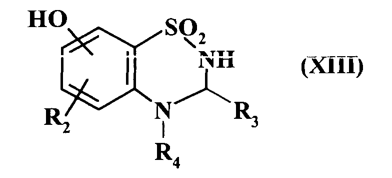 dans laquelle R₂, R₃ et R₄ ont la même signification que dans la formule (I),
composé de formule (VII) ou (XIII) que l'on fait réagir avec un dérivé d'acide boronique de formule **(XIV) :** 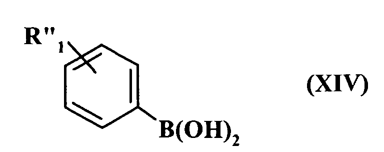 dans laquelle R"₁ représente un groupement cyano ou un hétérocycle,
pour conduire, (après transformation éventuelle du groupement R"₁ quand celui-ci représente un groupement cyano en groupement tétrazolyle correspondant), au composé de formule **(I/a**_{**1**}**)** ou **(I/a**_{**2**}**),** cas particuliers des composés de formule (I) : 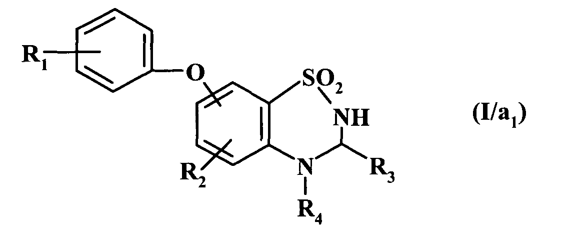 dans laquelle R₁, R₂, R₃ et R₄ ont la même signification que dans la formule (I), 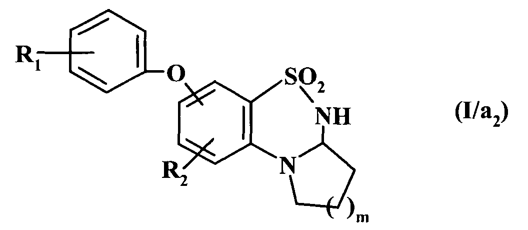 dans laquelle R₁, R₂ et m ont la même signification que dans la formule (I),
composés de formule (I/a₁) ou (I/a₂) :
que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

14. Procédé de préparation des composés de formule (I) selon la revendication 1 dans laquelle A représente un groupement CR₄R₅ **caractérisé en ce que** l'on utilise comme produit de départ, un composé de formule **(XV) :** 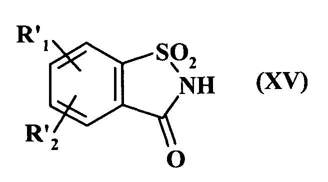 dans laquelle :
R'₁ représente un groupement alkoxy (C₁-C₆) linéaire ou ramifié,
R'₂ représente un atome d'hydrogène, d'halogène ou un groupement alkoxy (C₁-C₆) linéaire
ou ramifié,
qui subit l'action de la chloroacétone en présence de diméthylformamide, pour conduire au composé de formule **(XVI) :** 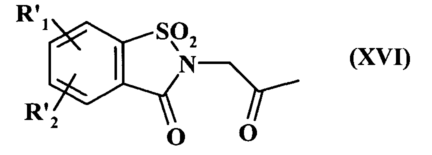 dans laquelle R'₁ et R'₂ ont la même signification que précédemment,
qui subit un réarrangement en milieu basique, pour conduire au composé de formule **(XVII)** : 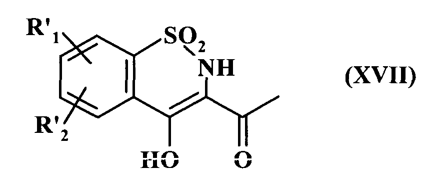 dans laquelle R'₁ et R'₂ ont la même signification que précédemment,
qui est déacétylé par chauffage au reflux en milieu benzénique en présence d'un excès d'éthylèneglycol et d'une quantité catalytique d'acide *p*-toluène sulfonique, pour conduire au composé de formule **(XVIII) :** 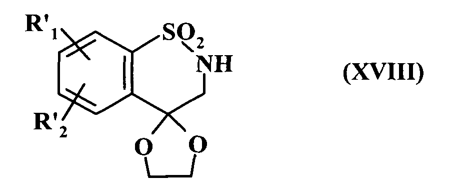 dans laquelle R'₁ et R'₂ ont la même signification que précédemment,
qui subit une hydrolyse en milieu acide, pour conduire au composé de formule **(XIXa) :** 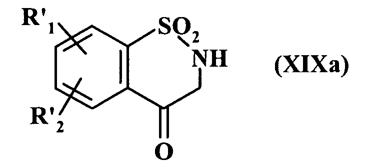 dans laquelle R'₁ et R'₂ ont la même signification que précédemment,
dont, éventuellement, selon la nature du groupement R₃ que l'on souhaite obtenir, on protège l'atome d'azote par un groupement protecteur, puis, après traitement par une base forte, que l'on traite par un composé de formule R'₃-P,
dans laquelle R'₃ représente un groupement alkyle (C₁-C₆) linéaire ou ramifié ou un groupement cycloalkyle (C₃-C₇) et P représente un groupe partant,
pour conduire après déprotection de l'atome d'azote, au composé de formule **(XIX'a) :** 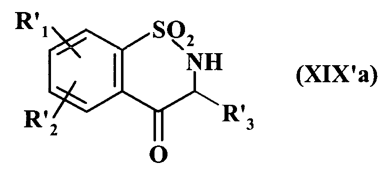 dans laquelle R'₁, R'₂ et R'₃ ont la même signification que précédemment,
composé de formule (XIXa) et (XIX'a) représentés par la formule **(XIX) :** 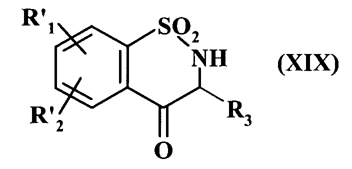 dans laquelle R'₁ et R'₂ ont la même signification que précédemment et R₃ est tel que défini dans la formule (I),
qui :
- **soit** subit une réduction catalytique, pour conduire au composé de formule **(XX)** : 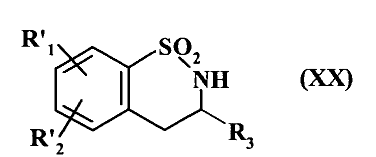 dans laquelle R'₁ et R'₂ ont la même signification que précédemment,
- **soit** est transformé par l'action d'un hydrure en alcool dont on transforme le groupement hydroxy en atome d'halogène par l'action d'un réactif approprié,
pour conduire au composé de formule **(XXI) :** 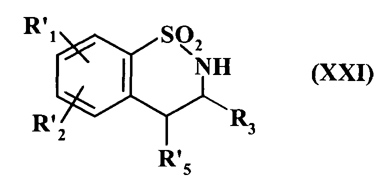 dans laquelle R'₁, R'₂ et R₃ ont la même signification que précédemment, et R'₅ représente un atome d'halogène ,
- **soit** subit l'action d'un organomagnésien R'₄MgBr dans lequel R'₄ représente un groupement alkyle (C₁-C₆) linéaire ou ramifié,
pour conduire au composé de formule **(XIXb) :** 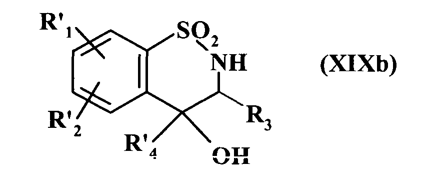 dans laquelle R'₁, R'₂, R₃ et R'₄ ont la même signification que précédemment,
composé de formule **(XIXb) :**
- **ou bien** qui subit une réduction catalytique, pour conduire au composé de formule **(XXII) :** 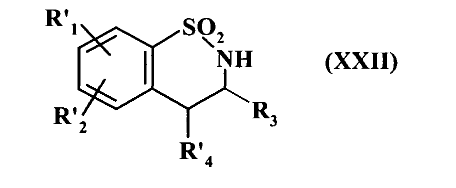 dans laquelle R'₁, R'₂, R₃ et R'₄ ont la même signification que précédemment,
- **ou bien** dont on transforme le groupement hydroxy en atome d'halogène par l'action d'un réactif approprié,
pour conduire au composé de formule **(XXIII)** : 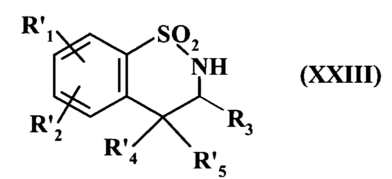 dans laquelle R'₁, R'₂, R₃ et R'₄ ont la même signification que précédemment et R'₅ représente un atome d'halogène,
composé de formule **(XX)** à **(XXIII)** dont on transforme le groupement R'₁ et le groupement R'₂ lorsque celui-ci représente un groupement alkoxy (C₁-C₆) linéaire ou ramifié, en groupements hydroxy,
pour conduire au composé de formule **(XXIV) :** 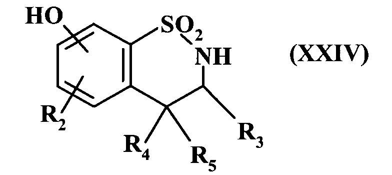 dans laquelle R₂. R₃, R₄ et R₅ ont la même signification que dans la formule (I),
composé de formule **(XXIV),**
que l'on fait réagir avec un dérivé d'acide boronique de formule **(XIV) :** 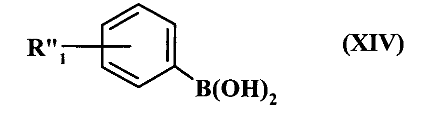 dans laquelle R"₁ représente un groupement cyano ou un hétérocycle,
pour conduire, (après transformation éventuelle du groupement R"₁ quand celui-ci représente un groupement cyano en groupement tétrazolyle correspondant), au composé de formule **(I/b),** cas particuliers des composés de formule (I) : 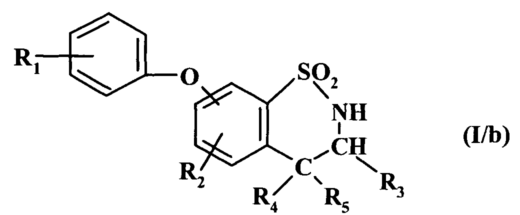 dans laquelle R₁, R₂, R₃, R₄, R₅ ont la même signification que dans la formule (I),
composé de formule (I/b), que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

15. Compositions pharmaceutiques contenant comme principe actif un composé selon l'une quelconque des revendications 1 à 12 en combinaison avec un ou plusieurs excipients ou véhicules inertes, non toxiques, pharmaceutiquement acceptables.

16. Compositions pharmaceutiques selon la revendication 15 contenant comme principe actif un composé selon l'une quelconque des revendications 1 à 12 utiles en tant que médicaments, comme modulateurs AMPA.
